# EUROPEAN PATENT APPLICATION

(11) **EP 2 644 593 A1**
(43) Date of publication of application: **02.10.2013**
(21) Application number: 12162417.5
(22) Date of filing: 30.03.2012
(51) Int. Cl.: C07C 251/76, C07C 69/716, C07D 231/14

(54) **Dialkoxymethyl oxobutyric acid esters, their manufacture and use**

(71) Applicant: SOLVAY SA, 1120 Bruxelles (BE)
(72) Inventor: Jaunzems, Janis, 30559 Hannover (DE)
(74) Representative: Mross, Stefan P.M.

(57) **Abstract**

Dialkoxymethyl oxobutyric acid esters of formula (I), R¹-C(O)-CH(R²)-C(O)-OR³, are provided, wherein R¹ denotes C1 to C5 alkyl ; C1 to C5 alkyl, substituted by at least one halogen atom ; R² denotes C(OR⁴)(OR⁵) wherein R⁴ and R⁵ are the same or different and denote C1 to C5 alkyl ; R3 denotes C1 to C6 alkyl ; C1 to C5 alkyl substituted by at least one halogen atom ; benzyl ; phenyl ; or allyl.

They can be manufactured from compounds of formula (II), R¹-C(O)-CH₂-C(O)-OR³, wherein R¹ and R³ have the meaning given above, and trialkylorthoformates.

They are useful, for example, as intermediates for the manufacture of fungicides.

## Description

The invention concerns dialkoxymethyl oxobutyric acid esters, a process for manufacturing them and their use as intermediates.

WO 2011/113789 discloses a process for the manufacture of alkyl 2-alkoxymethylene-4,4-difluoro-3-oxobutyrates. Such compounds are intermediate in organic synthesis and can be reacted with methyl hydrazine to provide, for example, 3-difluoromethyl-1-methyl-1H-pyrazole-4-carboxylic acid alkyl esters. Such pyrazoles are intermediates themselves and can be reacted with certain amines to provide fungicides such as Isopyrazam, Sedaxane, and others.

Object of the present invention is to provide intermediates suitable for the manufacture of pyrazole carboxylic acid esters. This object and other objects are achieved by the present invention as outlined in the description and the claims.

According to one aspect of the present invention, dialkoxymethyl oxobutyric acid esters of formula (I) are provided,

R¹-C(O)-CH(R²)-C(O)-OR³ (I)

wherein
R¹ denotes C1 to C5 alkyl; C1 to C5 alkyl, substituted by at least one halogen atom ;
R² denotes C(OR⁴)(OR⁵) wherein R⁴ and R⁵ are the same or different and denote C1 to C5 alkyl;
R3 denotes C1 to C6 alkyl; C1 to C5 alkyl substituted by at least one halogen atom ; benzyl ; phenyl ; or allyl.
R¹ preferably denotes C1 to C3 alkyl, substituted by at least one halogen atom.

More preferably, R1 is C1 to C3 alkyl, substituted by at least one F atom.

Still more preferably, R1 is C1 to C2 alkyl, substituted by at least two F atoms. Especially preferably, R1 is CF₃, CHF₂, CH₂F or CF₂Cl.

R³ is preferably C1 to C6 alkyl. More preferably, R³ is C1 to C3 alkyl. Especially preferably, R³ is methyl or ethyl.

R⁴ and R⁵ are preferably identical and denote methyl, ethyl or propyl, and especially denote ethyl.

Preferred compounds of formula (I) are compiled in the following table 1.

**Table 1 : Preferred compounds of formula (I)**

| |
|---|
| 4-chloro-4,4-difluoro-2-diethoxymethyl-3-oxo-butyric acid methyl ester |
| 4-chloro-4,4-difluoro-2-diethoxymethyl-3-oxo-butyric acid ethyl ester |
| 4-chloro-4,4-difluoro-2-diethoxymethyl-3-oxo-butyric acid n-propyl ester |
| 4,4,4-trifluoro-2-diethoxymethyl-3-oxo-butyric acid methyl ester |
| 4,4,4-trifluoro-2-diethoxymethyl-3-oxo-butyric acid ethyl ester |
| 4,4,4-trifluoro-2-diethoxymethyl-3-oxo-butyric acid n-propyl ester |
| 4,4-difluoro-2-diethoxymethyl-3-oxo-butyric acid methyl ester |
| 4,4-difluoro-2-diethoxymethyl-3-oxo-butyric acid ethyl ester |
| 4,4-difluoro-2-diethoxymethyl-3-oxo-butyric acid n-propyl ester |

In the following, the manufacture of the compounds of formula (I) is described.

The compounds of formula (I) can be manufactured from trialkyl orthoformiate and esters of 3-oxobutyric acid.

Accordingly, another aspect of the present invention concerns a process for the manufacture of compounds of formula (I) by reacting compounds of formula (II), R¹-C(O)-CH₂-C(O)-OR³, wherein R¹ and R³ have the meaning given above, and trialkylorthoformates of formula (III), HC(OR⁴)(OR⁵)(OR⁶); the groups R⁴, R⁵ and R⁶ may be the same or different and are methyl, ethyl or propopyl. Preferably, they are the same and are methyl, ethyl or n-propyl. Especially preferably, they are the same and are ethyl. Compounds of formula (II) can be manufactured by the addition of acetic acid chlorides to ketene as described in DE-AS 1158490.

The respective chemical reaction between compounds of formula (II) and formula (III) is assumed to be

R¹-C(O)-CH₂-C(O)-OR³ (II)

+

HC(OR⁴)(OR⁵)(OR⁶) → R¹-C(O)-CH(R²)-C(O)-OR³ + R⁶OH (III)

The reaction is performed at a temperature which allows for a reasonably fast reaction. Preferably, the temperature is equal to or greater than 80°C. More preferably, it is equal to or greater than 90°C. Especially preferably, it is equal to or greater than 100°C.

The upper limit of the reaction temperature is selected such that no undesired amounts of side reactions take place. Often, the reaction is performed at a temperature equal to or lower than 180°C, preferably, equal to or lower than 160°C.

If desired, the reaction can be performed in the presence of an inert gas, e.g. in the presence of N₂.

Contrary to reactions between compounds of formula (I) and (II) as described in the state of the art, no carboxylic acid anhydrides, for example, no acetic acid anhydride, are applied in the method of the invention.

If desired, the reaction can be performed in the presence of one or more solvents, for example, in the presence of at least one solvent selected from the group consisting of aprotic or protic organic solvents, e.g. esters, ethers or alcohols. According to a preferred embodiment, an excess of the compound of formula (III) is applied as solvent. Especially preferably, triethylorthoformate is the preferred compound of formula (III), and thus, it is the especially preferred solvent.

The alcohol which is formed during the reaction is preferably removed from the reaction mixture during the course of the reaction, e.g. by simple distillation.

The reaction is not sensitive to pressure ; it can be performed in a vacuum, at ambient pressure and under pressure above ambient pressure, e.g. at a pressure up to 10 bar (abs) or more. Since, in a preferred embodiment, the resulting alcohol is distilled off, performing the reaction at ambient pressure or applying a vacuum is preferred. The distilled alcohol is of such a high purity that it can be used for other useful purposes, e.g. as a solvent or as a reagent to produce compounds of formula (II) according to the description of DE-AS 1158490.

After completion of the reaction, preferably, any solvent is removed from the reaction mixture. This is preferably performed by applying a vacuum. If, for example, triethyl orthoformate ("TEOF") is applied as starting compound of formula (III), it is preferred to remove the excess of TEOF by keeping the reaction mixture at approximately 100°C and an initial vacuum of 100 mbar.

Solvent, e.g. trialkyl orthoformate when applied in excess, can be reused.

The resulting compound of formula (I) is obtained in sufficient purity for further reaction steps.

The compounds of formula (I) are especially suitable as intermediates in a process for the manufacture of pyrazoles or intermediates thereof. In such a type of reaction, the compound of formula (I) is reacted with an alkyl hydrazine, preferably methyl hydrazine. In a preferred mode of reaction, the compound of formula (I) is reacted with the intermediate of the reaction between an alkyl hydrazine and a ketone, especially between methyl hydrazine and acetone. As described in US 2011/0028735, an alkyl alkylidene hydrazone forms in this reaction ; for example, methyl hydrazine and acetone form methyl ethylidene hydrazone which is the most preferred reactant. From this intermediate, a pyrazole can be formed by cyclization initiated by an acid.

Accordingly, another aspect of the present invention concerns the use of the compounds of formula (I) in a process for the manufacture of compounds of formula (IV), R¹-C(O)-C(=CH-N(R⁶)-N=CR⁷R⁸-(O)-O-R³ wherein R¹ and R³ have the meaning given above, R⁶ is methyl, ethyl or propyl, preferably methyl, and R⁷ and R⁸ are the same or different and denote methyl, ethyl or propyl, preferably both R⁷ and R⁸ are methyl, by reaction of a compound of formula (I), R¹-C(O)-CH(R²)-C(O)-OR³ wherein R¹, R² and R³ have the meaning given above, with an alkyl hydrazine of formula (V), NH₂=NH(R⁶) in the presence of a ketone of formula (VI), R⁷R⁸C(O) wherein R⁶, R⁷ and R⁸ have the meaning given above, in a first step, and optionally, but preferably, further comprising a second step of cyclization of the compound of formula (IV) to form a pyrazole of formula (VII), 3-R¹-1-R⁶-1 H-pyrazole-4-COOR³ wherein R¹, R⁶ and R³ have the meaning given above. The preferred additional step of cyclization of the intermediate compound of formula (IV) can be initiated by acid, e.g. by adding HCl or hydrochloric acid in catalytic amounts.

Thus, the overall reaction to form a pyrazole proceeds via a first reaction step forming a alkanylidene intermediate which is then by cyclization in a second step provides the pyrazole.

Preferred groups R¹, R³ and R⁶ are given above. Especially preferably, the group R₁ in the pyrazole is CF₃, CF₂H, CFH₂ or CF₂Cl. R³ is preferably methyl, ethyl or propyl. R⁶ preferably is methyl.

In the first reaction step, the molar ratio between the compound of formula (I) and the hydrazine is preferably approximately 1:1 ± 0.1 ; more preferably, it is approximately 1:1. The temperature during the first reaction step is not critical, but is preferably from -10°C to + 50°C. The temperature during the second step is preferably between -10°C to + 50°C. The reaction is often exothermic, thus cooling of the reaction mixture is advantageous. Often, the cyclization reaction is performed at room temperature.

In following table 2, the most preferred intermediates of formula (IV) are compiled.

**Table 2 : Preferred compounds of formula (IV)**

| |
|---|
| CF₃-C(O)-C(=CH-N(CH₃)-N=C(CH₃)₂-(O)-O-CH₃ |
| CF₂Cl-C(O)-C(=CH-N(CH₃)-N=C(CH₃)₂-(O)-O-CH₃ |
| CHF₂-C(O)-C(=CH-N(CH₃)-N=C(CH₃)₂-(O)-O-CH₃ |
| CF₃-C(O)-C(=CH-N(CH₃)-N=C(CH₃)₂-(O)-O-CH₂CH₃ |
| CF₂Cl-C(O)-C(=CH-N(CH₃)-N=C(CH₃)₂-(O)-O-CH₂CH₃ |
| CHF₂-C(O)-C(=CH-N(CH₃)-N=C(CH₃)₂-(O)-O-CH₂CH₃ |
| CF₃-C(O)-C(=CH-N(CH₃)-N=C(CH₃)₂-(O)-O-CH₂CH₂CH₃ |
| CF₂Cl-C(O)-C(=CH-N(CH₃)-N=C(CH₃)₂-(O)-O-CH₂CH₂CH₃ |
| CHF₂-C(O)-C(=CH-N(CH₃)-N=C(CH₃)₂-(O)-O-CH₂CH₂CH₃ |

In following table 3, the most preferred pyrazole compounds of formula (VII) are compiled.

**Table 3 : Preferred pyrazole compounds**

| |
|---|
| 3-CF₃-1-CH₃-1 H-pyrazole-4-COOCH₃ |
| 3-CF₂Cl-1-CH₃-1 H-pyrazole-4-COOCH₃ |
| 3-CHF₂-1-CH₃-1 H-pyrazole-4-COOCH₃ |
| 3-CF₃-1-CH₃-1 H-pyrazole-4-COOCH₂CH₃ |
| 3-CF₂Cl-1-CH₃-1 H-pyrazole-4-COOCH₂CH₃ |
| 3-CHF₂-1-CH₃-1 H-pyrazole-4-COOCH₂CH₃ |
| 3-CF₃-1-CH₃-1 H-pyrazole-4-COOCH₂CH₂CH₃ |
| 3-CF₂Cl-1-CH₃-1 H-pyrazole-4-COOCH₂CH₂CH₃ |
| 3-CHF₂-1-CH₃-1 H-pyrazole-4-COOCH₂CH₂CH₃ |

It is an advantage of the present invention that pyrazole compounds can be manufactured in high selectivity. The formation of the respective 5-alkyl pyrazoles ("iso compounds") is very low.

Should the disclosure of any patents, patent applications, and publications which are incorporated herein by reference conflict with the description of the present application to the extent that it may render a term unclear, the present description shall take precedence.

The following examples are intended to describe the invention in further detail without the intention to limit it.

### General remarks :

Triethylorthoformate (TEOF) is commercially available. Ethyl difluorochloroacetic acid (ECDFAA) is commercially available from Solvay Fluor GmbH, Hannover /Deutschland.

### Example 1: Manufacture of 4-Chloro-2-diethoxymethyl-4,4-difluoro-3-oxobutyric acid ethyl ester ("ECDFAA-DEM").

TEOF (130 g, 0.88 mol) was placed in a flask equipped with a magnetic stirring bar and a vacuum distillation cooler. ECDFAA (35 g, 0.175 mol) was added.

The resulting mixture was heated to 110°C (outer temperature of the flask) under a mild vacuum (300 mbar) ; ethanol formed during the reaction is slowly distilled out of the vessel. After 2h, the starting material was completely consumed, and product formation was observed by GC analysis. Excess of TEOF was removed from the reaction mixture by distillation (at 100°C, starting with a pressure of 100 mbar at the beginning and 10 mbar at the end, to remove all TEOF traces), resulting pure product as pale yellow liquid with quantitative yield.

¹H NMR (500 MHz, CHLOROFORM-*d*) δ ppm 1.15 (t, 3 H) 1.20 (t, 3 H) 1.27 (t, 3 H) 3.49 - 3.57 (m, 1 H) 3.61 - 3.69 (m, 1 H) 3.71 - 3.79 (m, 2 H) 4.23 (q, 2 H) 4.34 (d, *J*=8.24 Hz, 1 H) 5.18 (d, *J*=8.24 Hz, 1 H).

### Example 2: Manufacture of Ethyl 4-chloro-4,4-difluoro-2-((1-methyl-2-(propan-2-ylidene)hydrazinyl)methylene)-3-oxobutanoate ("ECDFAA-MM")

To 25 ml of dry acetone, monomethyl hydrazine ("MMH") (0.76 g, 16.5 mmol) was added. A clear solution resulted which was stirred for 30 min at room temperature. Then, ECDFAA-DEM (5 g, 16.5 mmol) was added dropwise under moderate cooling with tap water. The resulting mixture was stirred for 15 h. A sample was taken from the reaction mixture and analyzed by GC which showed complete consumption of ECDFAA-DEM to form ECDFAA-MM. The crude yellow solution was entered directly into the next stage to form the respective pyrazole.

¹H NMR (500 MHz, CHLOROFORM-*d*) δ ppm 1.30 (bt, 3 H) 1.98 (s, 3 H) 2.10 (s, 3 H) 3.2 - 3.4 (bs, 3 H) 4.10 - 4.25 (bm, 2 H) 7.58 - 7.68 (bs, 1 H).

### Example 3: Manufacture of ethyl 3-(chlorodifluoromethyl)-1-methyl-1H-pyrazole-4-carboxylate ("CDFMMP")

To the crude solution obtained in example 2, 0.5 ml of 10 % HCl were added under moderate cooling with tap water. The resulting mixture was stirred for 30 min, and then the solvent was evaporated yielding almost pure CDFMMP as pale yellow oil.

¹H NMR (500 MHz, CHLOROFORM-*d*) δ ppm 1.35 (t, 3 H) 3.96 (s, 3 H) 4.32 (q, 2 H) 7.95 (s, 1 H).

## Claims

1. Compounds of formula (I),
R¹-C(O)-CH(R²)-C(O)-OR³ (I)
wherein
R¹ denotes C1 to C5 alkyl; C1 to C5 alkyl, substituted by at least one halogen atom ;
R² denotes C(OR⁴)(OR⁵) wherein R⁴ and R⁵ are the same or different and denote C1 to C5 alkyl;
R3 denotes C1 to C6 alkyl; C1 to C5 alkyl substituted by at least one halogen atom ; benzyl ; phenyl ; or allyl.

2. The compounds of claim 1 wherein R¹ denotes C1 to C3 alkyl, substituted by at least one halogen atom.

3. The compounds of claim 1 wherein R¹ denotes R1 C1 to C2 alkyl, substituted by at least two F atoms.

4. The compounds of claim 3 wherein R¹ is CF₃, CF₂H, CFH₂ or CF₂Cl.

5. The compounds of anyone of claims 1 to 4 wherein R⁴ and R⁵ are the same and denote methyl, ethyl or propyl.

6. The compounds of claim 5 wherein R⁴ and R⁵ denote ethyl.

7. The compounds of anyone of claims 1 to 6 wherein R³ is C1 to C3 alkyl.

8. The compounds of claim 1 which is 4-chloro-4,4-difluoro-2-diethoxymethyl-3-oxo-butyric acid methyl ester, 4-chloro-4,4-difluoro-2-diethoxymethyl-3-oxo-butyric acid ethyl ester, 4-chloro-4,4-difluoro-2-diethoxymethyl-3-oxo-butyric acid n-propyl ester, 4,4,4-trifluoro-2-diethoxymethyl-3-oxo-butyric acid methyl ester, 4,4,4-trifluoro-2-diethoxymethyl-3-oxo-butyric acid ethyl ester, 4,4,4-trifluoro-2-diethoxymethyl-3-oxo-butyric acid n-propyl ester, 4,4-difluoro-2-diethoxymethyl-3-oxo-butyric acid methyl ester, 4,4-difluoro-2-diethoxymethyl-3-oxo-butyric acid ethyl ester, or 4,4-difluoro-2-diethoxymethyl-3-oxo-butyric acid n-propyl ester.

9. A process for the manufacture of compounds of formula (I),
R¹-C(O)-CH(R²)-C(O)-OR³ (I)
wherein
R¹ denotes C1 to C5 alkyl; C1 to C5 alkyl, substituted by at least one halogen atom ;
R² denotes C(OR⁴)(OR⁵) wherein R⁴ and R⁵ are the same or different and denote C1 to C5 alkyl;
R3 denotes C1 to C6 alkyl; C1 to C5 alkyl substituted by at least one halogen atom ; benzyl ; phenyl ; or allyl,
by reacting a compound of formula (II), R¹-C(O)-CH₂-C(O)-OR³ wherein R¹, R² and R³ have the meaning given above, with a trialkylorthoformate of formula (III), HC(OR⁴)(OR⁵)(OR⁶), wherein R⁴, R⁵ and R⁶ are the same or different and denote methyl, ethyl or propyl.

10. The process of claim 9 wherein R¹ denotes C1 to C3 alkyl, substituted by at least one halogen atom ; wherein R⁴ and R⁵ are the same and denote methyl, ethyl or propyl ; and wherein R³ is C1 to C3 alkyl.

11. The process of claims 9 or 10 which is performed in the absence of carboxylic acid anhydrides.

12. Use of the compounds of formula (I) in a process for the manufacture of fungicides.

13. Use according to claim 12 wherein, in a first step, compounds of formula (IV) R¹-C(O)-C(=CH-N(R⁶)-N=CR⁷R⁸-(O)-O-R³ are manufactured, wherein R¹ denotes C1 to C5 alkyl; C1 to C5 alkyl, substituted by at least one halogen atom ; R³, denotes C1 to C6 alkyl; C1 to C5 alkyl substituted by at least one halogen atom ; benzyl ; phenyl ; or allyl, R⁶ is methyl, ethyl or propyl, and R⁷ and R⁸ are the same or different and denote methyl, ethyl or propyl.

14. Use according to claim 12 or 13 wherein, in a second step, pyrazoles of formula (VII), 3-R¹-1-R⁶-1 H-pyrazole-4-COOR³, are provided wherein R¹, R³ and R⁶ have the meaning given above.
